# EUROPEAN PATENT APPLICATION

(11) **EP 4 484 023 A1**
(43) Date of publication of application: **01.01.2025**
(21) Application number: 24183402.7
(22) Date of filing: 20.06.2024
(51) Int. Cl.: B05C 17/01

(54) **BULK DISPENSING DEVICE FOR SEALING COMPOUNDS**

(30) Priority: 22.06.2023 IN 202311041666
(71) Applicant: Eaton Intelligent Power Limited, Dublin 4 (IE)
(72) Inventor: Thorat, Hemraj K., 411036 Pune (IN); Kumar, Amresh, 841418 Saran (IN); Lopez, Joseph V., Camillus, 13031 (US); Kenyon, Timothy, Syracuse, 13208 (US); Maita Maita, Jessica M., Liverpool, 13088 (US)
(74) Representative: Schwan Schorer & Partner mbB

(57) **Abstract**

A system for bulk dispensing an expandable sealant for use in an electrical fitting configured to be used in a hazardous or harsh environment. The system includes a container having a first chamber for holding a first component and a second chamber for holding a second component. An injector is configured to receive the container. The injector includes a first plunger and a second plunger wherein the first plunger aligns with the first chamber and the second chamber aligns with the second plunger when the container is positioned in the injector. A nozzle is configured to engage a distal end of the container. The nozzle is positioned to fluidly communicate with the first chamber and the second chamber when attached to the container. The nozzle has an internal labyrinth passage configured to fluidly connect to the first chamber and the second chamber.

## Description

### CROSS REFERENCE TO RELATED APPLICATIONS

This application claims benefit of and priority to Indian Patent Application No. 202311041666, filed June 22, 2023, entitled "BULK DISPENSING DEVICE FOR SEALING COMPOUNDS," which is incorporated herein in its entirety.

### Field

The subject application relates to dispensing of sealing compounds. More particularly, an apparatus for dispensing sealing compounds used in electrical connections for hazardous or harsh conditions.

### Background

Sealing compounds are used to limit the passage of vapor and/or flames through a sealing fitting. The use of sealing compounds allows the sealing fitting to be used in hazardous locations where the passage of these vapors and/or flames through the sealing fitting is undesirable.

Some sealing compounds require that a user mixes a powder with water, stir the mixture and then pour the mixture into the sealing fitting. In some instances it is difficult to maintain the sealing compound in the appropriate parts of the sealing fitting as the sealing compound cures. This challenge is increased because of the long cure time of the sealing compound, i.e., typically on the order of 8 hours or more for powder-type compounds.

To aid in placing the sealing compound properly in the sealing fitting, fiber dams may be placed in the sealing fitting before the sealing compound is inserted. The fiber dam helps to prevent the sealing compound from draining out of the sealing fitting during the curing process.

The present application provides an apparatus to improve the placement and curing of a sealing compound in a sealing fitting.

### Summary of the Invention

There is provided a system for bulk dispensing an expandable sealant for use in an electrical fitting configured to be used in a hazardous or harsh environment. The system includes a container having a first chamber for holding a first component and a second chamber for holding a second component. The first chamber is fluidly isolated from the second chamber. An injector is configured to receive the container. The injector includes a first plunger and a second plunger wherein the first plunger aligns with the first chamber and the second chamber aligns with the second plunger when the container is positioned in the injector. A nozzle is configured to engage a distal end of the container. The nozzle is positioned to fluidly communicate with the first chamber and the second chamber when attached to the container. The nozzle has an internal labyrinth passage configured to fluidly connect to the first chamber and the second chamber. Actuation of the injector causes the first plunger and the second plunger to force the first component and the second component out of the container and into the nozzle wherein the first component and the second component are mixed to form the expandable sealant for injection into the electrical fitting.

In the foregoing system, a meter is provided for dispensing predetermined volumes of the first component and the second component.

In the foregoing system, the injector includes a trigger for actuating the first plunger and the second plunger.

In the foregoing system, the trigger is indexed to incrementally displace the first plunger and the second plunger.

In the foregoing system, a cap is configured to engage the container to seal both the first chamber and the second chamber when the nozzle is disconnected from the container.

In the foregoing system, a first valve is attached to the first chamber and a second valve is attached to the second chamber. The first valve and the second valve are configured to be normally closed and to open when the first chamber and the second chamber, respectively, reach a predetermined internal pressure.

There is further provided a method for dispensing an expandable sealant from a container having a first chamber for holding a first component and a second chamber for holding a second component therein. The first chamber is fluidly isolated from the second chamber. The method includes: attaching the container to an injector, the injector including a first plunger and a second plunger wherein the first plunger aligns with the first chamber and the second plunger aligns with the second chamber when the container is positioned in the injector; attaching a nozzle to a distal end of the container, the nozzle positioned to fluidly communicate with the first chamber and the second chamber when attached to the container, the nozzle having an internal labyrinth passage configured to fluidly connect to the first chamber and the second chamber; and actuating the injector to cause the first plunger and the second plunger to force the first component and the second component out of the container and into the nozzle wherein the first component and the second component are mixed to form an expandable sealant for injection into an electrical fitting used in a hazardous or harsh environment.

In the foregoing method, the step of actuating the injector includes a meter for dispensing predetermined volumes of the first component and the second component.

In the foregoing method, the step of actuating the injector includes actuating a trigger for moving the first plunger and the second plunger.

In the foregoing method, the trigger is indexed to incrementally add predetermined volumes of the first component and the second component.

In the foregoing method, a step is provided of removing the nozzle and applying a cap to the container, wherein the cap is configured to seal both the first chamber and the second chamber.

In the foregoing method, the container includes a first valve attached to the first chamber and a second valve attached to the second chamber. The first valve and the second valve are configured to be normally closed and to open when the first chamber and the second chamber, respectively, reach a predetermined internal pressure.

### Brief Description of the Drawings

FIG. 1 is front perspective view of a sealing fitting;
FIG. 2 is a side view of an injector with a container attached thereto;
FIG. 3 is an enlarged view of the container of FIG. 2; and;
FIG. 4 is a side view of nozzles configured to attach to the container of FIG. 3.

### Detailed Description

The following presents a description of the disclosure; however, aspects may be embodied in many different forms and should not be construed as limited to the embodiments set forth herein. Furthermore, the following examples may be provided alone or in combination with one or any combination of the examples discussed herein.

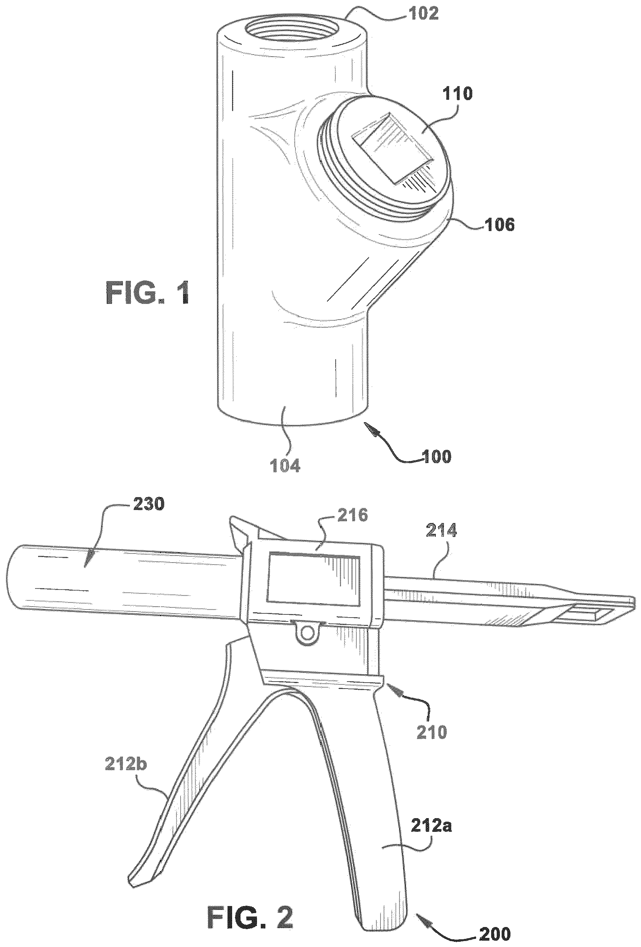

FIG. 1 illustrates a fitting 100 orientated in a vertical direction, i.e., a major axis of the fitting 100 extends vertically. The fitting 100 includes a first open end 102 and a second open end 104. The first open end 102 includes threads for allowing a conduit (not shown) to be threaded on the first open end 102. The second open end 104 also includes threads (not shown) for allowing a conduit (not shown) to be threaded on the second open end 104.

The fitting 100 is configured to join two conduits (not shown) together such that conductors (not shown) may extend from one conduit to the other conduit. The fitting 100 includes an angled opening 106 that is formed in a body of the fitting 100 between the first open end 102 and the second open end 104. A plug 110 is provided to be threaded into the angled opening 106 to seal the angled opening 106.

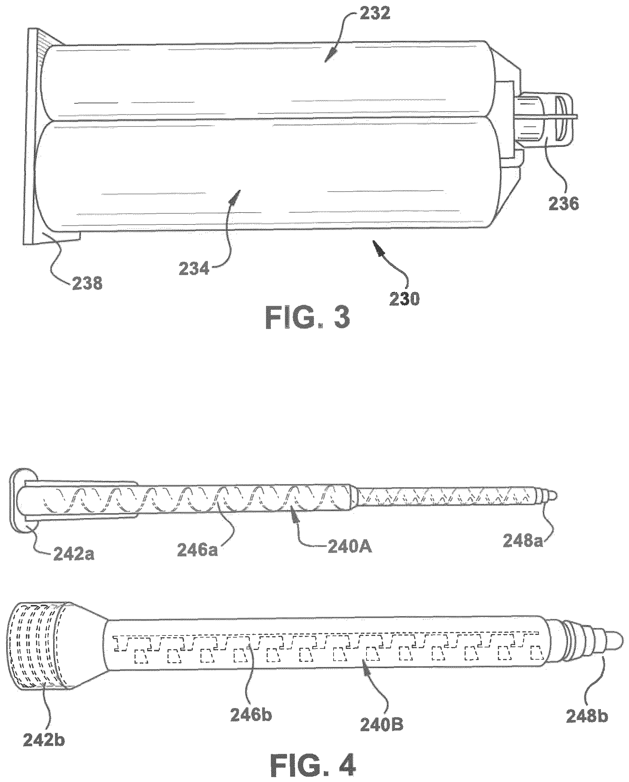

Referring to FIGS. 2-4, there is provided a dispensing assembly 200 for injecting a sealant into the angled opening 106. The dispensing assembly 200 includes an injector 210, a twin tube cartridge 230 and nozzles 240A, 240B (FIG. 4).

Referring to FIG. 2, the injector 210 includes a handle 212a and a trigger 212b. The trigger 212b is configured to move a plunger 214 of the injector 210 when actuated by a user, i.e., when the trigger 212b and handle 212a are squeezed by the user so that the trigger 212b moves toward the handle 212a. It is contemplated that the trigger 212b may include detents that allow the trigger 212b to incrementally move the plunger 214 in a stepwise manner when actuated. A housing 216 of the injector 210 is configured to receive the twin tube cartridge 230. It is contemplated that the housing 216 may include a slot (not shown) that is dimensioned to engage a mating feature (discussed in detail below) on an distal end of the twin tube cartridge 230.

Referring to FIG. 3, the twin tube cartridge 230 includes two tubes or chambers. i.e., a first tube or chamber 232 and a second tube or chamber 234 that are fluidly isolated from each other. The first tube 232 holds a first compound and the second tube 234 holds a second compound. The first compound and the second compound are selected so that they are non-reactive until they are mixed together. A first valve (not shown) is provided proximate a dispensing port of the first tube 232 and a second valve (not shown) is provided proximate a dispensing port of the second tube 234. The first and second valves are normally closed valves that are dimensioned to open when the pressure in the first tube or chamber 232 and the second tube or chamber 234, respectively, reach a predetermined internal pressure. Once the pressure is removed, the first and second valves return to the closed position. The twin tube cartridge 230 includes an outlet port 236 that is fluidly connected to the first tube or chamber 232 and the second tube or chamber 234. An opposite end of the twin tube cartridge 230 includes a mating flange 238 that extends outwardly from the end of the twin tube cartridge 230. The flange 238 is dimensioned to be received in the slot (not shown) on the housing 216 of the injector 210 to secure the twin tube cartridge 230 to the injector 210.

Referring to FIG. 4, the nozzles 240A, 240B are configured to attach to the dispensing ports of the twin tube cartridge 230. The nozzle 240A includes an inlet port 242a that leads to a labyrinth passage 246a wherein two fluids entering the inlet port 242a are mixed together as they travel from the inlet port 242a to an outlet port 248a. Similarly, the nozzle 240B includes an inlet port 242b that leads to a labyrinth passage 246b wherein two fluids entering the inlet port 242b are mixed together as they travel from the inlet port 242b to an outlet port 248b. In the embodiments illustrated, the inlet port 242a of the nozzle 240A includes a flange that is dimensioned to engage the outlet port 236 of the twin tube cartridge 230. The flange may be secured to the outlet port 236 by twisting the flange into a mating slot (not shown) formed in the outlet port 236. The inlet port 242b of the nozzle 240B may have a thread that is dimensioned to be threaded onto a mating thread on the outlet port 236 of the twin tube cartridge 230 to secure the nozzle 240B to the twin tube cartridge 230.

The present invention will now be described with respect to the operation of the same. Referring to FIG. 2, the twin tube cartridge 230 is attached to the housing 2 1 6 of the injector 210 such that each tube 232, 234 aligns with a respective plunger 214 of the injector 210. One of the nozzles 240A, 240B is attached to the distal end of the twin tube cartridge 230. As the nozzle 240A, 240B is secured to the twin tube cartridge 230, a fluid connection is established between the tubes 232, 234 and the labyrinth passage 246a, 246b of the nozzle 240A, 240B.

To dispense the fluids from the twin tube cartridge 230, the trigger 212b is actuated to cause the plungers 214 to move into the respective tubes 232, 234. In particular, the handle 212a and the trigger 212b are squeezed by the user so that the trigger 212b moves toward the handle 212a. As the trigger 212b moves toward the handle 212a, the plungers 214 move into the tube 232, 234 thereby increasing the pressure within the tubes 232, 234. As the pressure in the tubes 232, 234 increases, they reach a level that is sufficient to cause the valves in the twin tube cartridges 230 to move from the close position to the open position. Once the valves are opened, the components in the tubes 232, 234 are forced out of the tubes 232, 234, past the valves (not shown) and into the nozzle 240A, 240B. As the two components flow through the nozzle 240A, 240B, they are forced to mix with each other within the labyrinth passage 246a, 246b. As the components mix, a chemical reaction occurs to form a sealant. The sealant is then ejected out of the nozzle 240A, 240B and into the angled opening 106. Once the fitting 100 is filled to the proper level, the plug 110 is secured to the angled opening 106 to close it. After the desired sealant has been dispensed, the pressure on the trigger 212b is released such that the pressure in the tubes 232, 234 decreases to allow the valves to return to the closed position. A cap (not shown) may then be attached to the outlet port 236 of the twin tube cartridge 230 to seal off the tubes 232, 234 so the twin tube cartridge 230 may be used at a later time.

The sealant formed is designed to expand within the fitting 100 to fill voids within the fitting 100 and to separate the conductors from each other and the walls of the fitting 100. The ability for the sealant to expand in all directions reduces or eliminates the need for filter dams when the fitting 100 is in a horizontal orientation. Further, the curing time of the sealant is greatly reduced as compared to powdered-type compounds.

It is contemplated that a digital meter (not shown) may be attached to the injector 210 for metering the first component and the second component out of the twin tube cartridges 230. The digital meter may also be calibrated to dispense the correct volumes of the first component and the second component based on a size of the fitting 100. The calibration may help to increase the repeatability in forming a proper seal in each application.

It will be apparent to those skilled in the art that various modifications and variations can be made without departing from the spirit and scope of the claimed invention.

## Claims

1. A system for bulk dispensing an expandable sealant for use in an electrical fitting configured to be used in a hazardous or harsh environment, the system comprising:
a container having a first chamber for holding a first component and a second chamber for holding a second component, wherein the first chamber is fluidly isolated from the second chamber;
an injector configured to receive the container, the injector including a first plunger and a second plunger wherein the first plunger aligns with the first chamber and the second chamber aligns with the second plunger when the container is positioned in the injector; and
a nozzle configured to engage a distal end of the container, the nozzle positioned to fluidly communicate with the first chamber and the second chamber when attached to the container, the nozzle having an internal labyrinth passage configured to fluidly connect to the first chamber and the second chamber,
wherein actuation of the injector causes the first plunger and the second plunger to force the first component and the second component out of the container and into the nozzle wherein the first component and the second component are mixed to form the expandable sealant for injection into the electrical fitting.

2. The system of claim 1, further comprising a meter for dispensing predetermined volumes of the first component and the second component.

3. The system of claim 1, the injector comprising a trigger for actuating the first plunger and the second plunger.

4. The system of claim 3, wherein the trigger is indexed to incrementally displace the first plunger and the second plunger.

5. The system of claim 1, further comprising a cap configured to engage the container to seal both the first chamber and the second chamber when the nozzle is disconnected from the container.

6. The system of claim 1, further comprising a first valve attached to the first chamber and a second valve attached to the second chamber, the first valve and the second valve configured to be normally closed and to open when the first chamber and the second chamber, respectively, reach a predetermined internal pressure.

7. A method for dispensing an expandable sealant from a container having a first chamber for holding a first component and a second chamber for holding a second component, wherein the first chamber is fluidly isolated from the second chamber, the method comprising:
attaching the container to an injector, the injector including a first plunger and a second plunger wherein the first plunger aligns with the first chamber and the second plunger aligns with the second chamber when the container is positioned in the injector;
attaching a nozzle to a distal end of the container, the nozzle positioned to fluidly communicate with the first chamber and the second chamber when attached to the container, the nozzle having an internal labyrinth passage configured to fluidly connect to the first chamber and the second chamber; and
actuating the injector to cause the first plunger and the second plunger to force the first component and the second component out of the container and into the nozzle wherein the first component and the second component are mixed to form an expandable sealant for injection into an electrical fitting used in a hazardous or harsh environment.

8. The method of claim 7, wherein the step of actuating the injector includes a meter for dispensing predetermined volumes of the first component and the second component.

9. The method of claim 7, wherein the step of actuating the injector includes actuating a trigger for moving the first plunger and the second plunger.

10. The method of claim 9, wherein the trigger is indexed to incrementally add predetermined volumes of the first component and the second component.

11. The method of claim 7, further comprising a step of removing the nozzle and applying a cap to the container, wherein the cap is configured to seal both the first chamber and the second chamber.

12. The method of claim 7, wherein the container includes a first valve attached to the first chamber and a second valve attached to the second chamber, the first valve and the second valve configured to be normally closed and to open when the first chamber and the second chamber, respectively, reach a predetermined internal pressure.
